# FASCICULE DE BREVET EUROPEEN

(11) **EP 0 970 940 B1**
(45) Date de publication et mention de la délivrance du brevet: **24.10.2001**
(21) Numéro de dépôt: 99401718.4
(22) Date de dépôt: 08.07.1999
(51) Int. Cl.: C07C 39/04, C07C 15/44

(54) **Procédé de récupération de composés dans les produits lourds issus de leur fabrication**
Verfahren zur Rückgewinnung von Produkten enthalten in den Schwer-Nebenprodukten aus ihrer Herstellung
Process for the recovery of products contained in the heavy side products from their preparation

(30) Priorité: 10.07.1998 FR 9808935
(43) Date de publication de la demande: 12.01.2000
(73) Titulaire: RHODIA CHIMIE, 92408 Courbevoie Cédex (FR)
(72) Inventeur: Igersheim, Françoise, 69003 Lyon (FR); Charrin, Jean-Jacques, 69003 Lyon (FR); Queiroz, Antonio, 13026-430 Jd Paraiso Campinas, Sao Paulo (BR)
(74) Mandataire: Wattremez, Catherine

(56) Documents cités:
- EP-A- 0 770 591
- GB-A- 1 479 124

## Description

La présente invention a pour objet un procédé de récupération de composés présents dans un mélange de produits lourds ; ledit mélange de produits lourds étant issu d'un procédé de préparation de l'un des composés que l'on souhaite récupérer.

Limiter dans la mesure du possible les pertes en produits (réactifs/ produits finaux) a toujours été l'une des préoccupations essentielles des procédés industriels, que ce soit pour des raisons évidentes d'économie du procédé, mais aussi pour des raisons liées à l'environnement et pour éviter de trop multiplier les traitements des effluents ou des résidus produits.

Ce problème est d'autant plus aigu que le procédé conduit à la formation de produits lourds visqueux dans lesquels sont emprisonnés des quantités non négligeables en produits souhaités ou valorisables.

C'est notamment le cas lors de la fabrication du phénol à partir de cumène.

Il est précisé qu'au sens de l'invention, on entend par produits lourds, des produits dont la température d'ébullition est élevée. Dans le cas particulier de la réaction indiquée ci-dessus, ces produits lourds contiennent principalement des cumylphénols et des dimères de l'α-méthylstyrène.

Le procédé le plus courant de traitement des produits lourds issus de la fabrication du phénol, consiste à mettre en oeuvre une opération de craquage thermique de ces résidus. Plus précisément, il s'agit de mettre en oeuvre une réaction durant laquelle des molécules sont dissociées en produits souhaités ou valorisables, suivie par une distillation immédiate des produits obtenus lors de la dissociation. L'inconvénient de ce procédé réside dans le fait que les températures de mises en oeuvre relativement élevées (environ 320°C), sont la cause de l'apparition d'impuretés, telles que les crésols, qui sont difficiles à séparer et qui, par conséquent, vont polluer les composés récupérés.

On a alors proposé de mettre en oeuvre un catalyseur acide, comme l'acide phosphorique, l'acide sulfurique, ou bien encore des acides organiques sulfoniques. L'emploi du catalyseur permet de diminuer la température de la réaction et donc d'éviter l'apparition des impuretés polluantes (voir par exemple GB-A-1 479 124). Cependant, ce procédé catalytique présente l'inconvénient de rendre difficilement valorisables les résidus qui sont obtenus. D'une part, on a constaté que ces résidus, outre leur viscosité élevée, comprennent des solides dispersés, provenant de la présence du catalyseur, et des sels de sodium, ce qui contribue à rendre très complexe, voire impossible, leur manipulation. D'autre part, et cela constitue un inconvénient majeur, ces résidus solides prennent en masse très facilement et rapidement, rendant l'appareillage inutilisable au moins provisoirement. En effet, en cas de prise en masse, il est nécessaire de débarrasser l'appareil, des résidus solidifiés en employant des moyens de nettoyage mécaniques puissants.

Comme on peut le constater, on ne dispose pas à l'heure actuelle de procédés permettant de récupérer au mieux des composés contenus dans un mélange de produits lourds, tout en rendant possible, simple, et rentable, la valorisation des résidus obtenus (essentiellement destruction avec récupération de chaleur).

Ces buts et d'autres sont atteints par la présente invention qui a pour objet un procédé de récupération de composés contenus dans un mélange de produits lourds, dans lequel on met en oeuvre une réaction/distillation grâce à laquelle on obtient, d'une part, lesdits composés, et d'autre part, des résidus ; le procédé selon l'invention consistant à effectuer une dilacération des résidus, préalablement à leur destruction, et avant qu'ils ne prennent en masse.

On a constaté en effet que mettre en oeuvre une opération de dilacération des résidus obtenus après la réaction/distillation évitait la prise en masse dans l'appareillage, ou tout au moins permettait de retarder suffisamment l'apparition de ce phénomène ; le temps de transporter les résidus dans une chaudière ou tout autre appareil dans lequel ils seront brûlés.

Par ailleurs, le procédé selon l'invention permet de récupérer des quantités importantes de composés présents dans le mélange de produits lourds.

Mais d'autres avantages et caractéristiques de la présente invention apparaîtront plus clairement à la lecture de la description, des exemples qui vont suivre.

Ainsi, le procédé selon l'invention est lié à la récupération de composés contenus dans un mélange de produits lourds.

Tout d'abord, le mélange de produits lourds fait partie des produits synthétisés au cours de la réaction principale.

Si l'on prend l'exemple de la préparation de phénol à partir de cumène (réaction principale), consistant à décomposer intermédiairement l'hydroperoxyde de cumyle, il se forme, outre le phénol et l'acétone, des produits tels que l'α-méthylstyrène (valorisable), ainsi que d'autres produits dont le point d'ébullition est plus élevé que celui du phénol. Parmi de tels produits, figurent notamment les cumyl-phénols ainsi que des dimères de l'α-méthylstyrène.
C'est ce mélange comprenant les cumyl-phénols ainsi que les dimères de l'α-méthylstyrène, que l'on appelle mélange de produits lourds, dans le cas particulier de cette réaction. Il est par ailleurs à noter que ce mélange est alcalin, du fait des traitements antérieurs.

Le mélange directement obtenu après la réaction de décomposition de l'hydroperoxyde de cumyle est, de manière habituelle, traité afin de séparer principalement, le phénol et l'acétone. En général, on procède en mettant en oeuvre au moins une étape de distillation,

Le mélange de produits lourds selon la présente invention correspond à celui obtenu après avoir mis en oeuvre l'étape de séparation précitée.

L'opération ultérieure de réaction/distillation (ou craquage) conformément au procédé selon l'invention peut être réalisée avec ou sans catalyseur. De préférence, la réaction est effectuée en présence d'un catalyseur.

On utilise en général un catalyseur de type acide minéral ou acide organique. L'acide sulfurique est un exemple approprié d'acide minéral utilisable en tant que catalyseur. A titre d'exemple d'acides organiques, on peut notamment citer les acides organiques sulfoniques, et de préférence l'acide para-toluène sulfonique.

Selon un mode de réalisation préféré de l'invention, on met en oeuvre la réaction/distillation du mélange de produits lourds, en présence d'un acide organique, et plus particulièrement d'acide para-toluène sulfonique.

La quantité de catalyseur mise en oeuvre peut être déterminée sans difficulté par l'homme de l'art. A titre d'illustration, la quantité de catalyseur mise en oeuvre est telle que la concentration en acide, après la neutralisation de la base présente dans le mélange de produits lourds à traiter, est plus particulièrement 0,1 à 3 % en poids du flux de mélange à traiter.

De manière avantageuse, on met en contact le catalyseur en amont de l'appareillage dans lequel le traitement du mélange de produits lourds est réalisé.

Par ailleurs, et selon un mode de réalisation particulier du procédé selon l'invention, le mélange de produits lourds après la mise en contact avec le catalyseur, est tout d'abord homogénéisé.

Pour ce faire, on utilise tout type de mélangeur classique dans le domaine. De préférence, on effectue cette homogénéisation dans un mélangeur statique.

L'opération de traitement du mélange de produits lourds consiste donc en une réaction/distillation.

Celle-ci est réalisée dans un réacteur muni, avantageusement, de moyens d'agitation.

Ce réacteur est par ailleurs surmonté d'une colonne de distillation.

Selon les produits à séparer, elle présente un nombre de plateaux théoriques compris entre 3 et 10, de préférence compris entre 4 et 6.

La colonne peut comprendre un garnissage ordonné, des anneaux, des plateaux (perforés, clapets, cloches, etc.).

Le procédé selon l'invention est mis en oeuvre dans des conditions telles que la température de réaction reste comprise entre 200 et 300°C, de préférence entre 220 et 260°C. Ces températures sont données à pression atmosphérique.

Bien évidemment, on ne sortirait pas du cadre de la présente invention en réalisant cette opération sous pression réduite, ou bien sous une légère surpression, auquel cas la température serait adaptée en conséquence.

En tête de la colonne de distillation sont récupérés les composés valorisables.

Ainsi, le procédé selon l'invention permet de récupérer non seulement du phénol par craquage des cumyl-phénols présents dans le mélange de produits lourds, mais aussi l'α-méthylstyrène, par craquage des cumyl-phénols et par craquage du dimère de l'α-méthylstyrène.

Après condensation desdits composés, une partie est recyclée à la colonne pour constituer le reflux, l'autre est séparée pour être stockée, ou encore faire l'objet de traitements ultérieurs en vue de séparer les divers constituants.

D'une manière très avantageuse, on récupère des composés purs. Ainsi, le phénol récupéré selon le procédé de l'invention est essentiellement exempt de crésols.

Une variante de la présente invention consiste, une fois les composés récupérés, à les additionner d'une base de manière à neutraliser l'acidité résiduelle due à l'utilisation du catalyseur. On a en effet constaté que la présence d'espèces comme SO₃ et/ou SO₂, pouvait causer une perte en composés récupérés, à cause de réactions de condensation inverse des réactions réalisées lors de l'étape de réaction/distillation.

Afin d'éviter ou de limiter dans la mesure du possible, toute production importante de mousse, il peut être avantageux d'introduire dans l'appareil où est effectuée la réaction/distillation des produits lourds, au moins un agent anti-mousse.

Plus particulièrement, on met en oeuvre des anti-mousses à base de silicones, résistant dans les conditions de mise en oeuvre de la réaction/distillation, tels que des produits de la gamme Rhodorsil® (commercialisé par Rhodia Chimie).

L'introduction de cet agent anti-mousse a de préférence lieu directement dans l'appareil où sont traités les résidus.

La quantité varie dans de larges limites et peut être définie sans difficulté par l'homme de l'art.

Comme cela a été indiqué plus haut, le procédé selon l'invention consiste tout particulièrement à dilacérer les résidus ayant subi l'opération de réaction/distillation qui vient d'être décrite.

Plus particulièrement, on dilacère les résidus dès la sortie de la réaction/distillation.

Plus précisément, si le procédé selon l'invention est mis en oeuvre en discontinu, alors l'opération de dilacération est mise en oeuvre dès la fin de la réaction/distillation.

Dans le cas où le procédé selon l'invention est mis en oeuvre en continu, l'opération de dilacération est mise en oeuvre sur les résidus, directement, sans période de stockage intermédiaire, et dès qu'ils sont soutirés de l'appareil dans lequel a lieu la réaction/distillation. Cela permet d'éviter toute prise en masse intermédiaire dudit mélange de composés.

Précisons que le débit alimenté en mélange de produits lourds, et la somme des débits soutirés en composés et en résidus, sont adaptés de manière à avoir une installation dont le fonctionnement est stable en continu.

Un autre avantage de la présente invention est qu'elle est tout à fait convenable pour un traitement en continu.

Cette opération de dilacération conforme à l'invention, est mise en oeuvre au moyen d'un appareil permettant de diminuer la taille des particules en suspension, de les homogénéiser et de les transporter. Les pompes dilacératrices conviennent particulièrement à la mise en oeuvre de l'invention.

Ces pompes sont bien connues par l'homme du métier. Elles sont notamment commercialisées par les sociétés Moritz, Mouvex, Baudot-Hardol.

A titre d'illustration, les pompes dilacératrices peuvent comprendre un rotor. généralement incliné par rapport à l'axe de rotation, et muni de préférence de moyens de broyage (comme des dents par exemple). Quant au stator, il possède habituellement des segments rainurés.

Ainsi, les résidus sont soutirés du réacteur dans lequel a lieu la réaction/distillation, pour être alimentés dans une cuve munie d'une telle pompe.

On ne sortirait pas du cadre de la présente invention en ajoutant aux résidus, avant l'opération de dilacération, un mélange dont le point d'ébullition est supérieur à la température d'ébullition des résidus. Par exemple, il peut être avantageux d'ajouter les composés à point d'ébullition élevé, issus de la préparation du cumène.

Les résidus, issus du procédé selon l'invention, éventuellement additionnés d'un mélange présentant un point d'ébullition important, sont donc ensuite dilacérés avant d'être détruits par combustion avec récupération des calories.

Il est à noter que pour des raisons de corrosivité de ces résidus, il est avantageux de neutraliser ces derniers, après l'opération de dilacération, en y incorporant une base. Par exemple, on peut utiliser les hydroxydes de métaux alcalins.

Précisons aussi qu'il est tout à fait envisageable d'ajouter, s'ils sont présents sur le site industriel, des composés basiques destinés à être détruits par la suite. Bien évidemment cela est possible dans la mesure où ces composés basiques ne réagissent pas avec les résidus pour donner des produits insolubles susceptibles de causer des problèmes de mise en oeuvre (bouchage).

De manière évidente, les matériaux employés pour l'appareillage sont choisis de manière à pouvoir résister à la corrosivité des résidus traités, et cela d'autant plus que l'on met en oeuvre un catalyseur pour effectuer l'étape de réaction/distillation.

Des exemples concrets vont maintenant être présentés.

### EXEMPLE

1/ On alimente dans un mélangeur statique 750 kg/h de résidus, de composition suivante :
   - environ 40 % de cumyl-phénols,
   - environ 20 % de dimères d'α-méthylstyrène,
   - environ 2 % de phénol,
   - qsp 100 % en autres produits lourds.

   On ajoute simultanément 15 kg/h d'acide paratoluène-sulfonique (solution aqueuse à 65 %).
2/ Le mélange résultant est introduit dans un réacteur muni de moyens d'agitation, et chauffé à une température de 250 °C.
   Le réacteur est surmonté par une colonne à distiller à 8 plateaux à cloches.
   Le taux de reflux est de 50 %.
   Le temps de séjour des résidus dans le réacteur, est d'environ 3 heures.
3/ On récupère en tête de la colonne à distiller, un flux de 375 kg/h comprenant :
   - environ 33 % de phénol
   - environ 40 % d'α-méthylstyrène
   - environ 15 % de cumène
   - qsp 100 % en produits divers non dosés.

   On ajoute au flux de ces composés de l'hydroxyde de sodium pour neutraliser l'acidité résiduelle.
4/ On soutire du réacteur, 375 kg/h de résidus, qui sont mélangés avec 200 kg/h d'un mélange de composés dont le point d'ébullition est supérieur à 250°C issus de la préparation du cumène.

Ce mélange est dilacéré au moyen d'une pompe de type Moritz ® (pompe dilacératrice) et enfin incinérés sans stockage intermédiaire.

### EXEMPLE COMPARATIF

On réalise le même essai en remplaçant la pompe dilacératrice par une pompe centrifuge classique.

On observe rapidement un bouchage de la ligne d'envoi à l'incinération.

## Revendications

1. Procédé de récupération de composés contenus dans un mélange de produits lourds, procédé dans lequel on met en oeuvre une réaction/distillation grâce à laquelle on obtient, d'une part, lesdits composés, et d'autre part, des résidus, **caractérisé en ce que** l'on effectue une dilacération des résidus, préalablement à leur destruction, et avant qu'ils ne prennent en masse.

2. Procédé selon la revendication précédente, **caractérisé en ce que** l'on effectue l'opération de dilacération dès la sortie de la réaction/distillation.

3. Procédé selon l'une quelconque des revendications précédentes, **caractérisé en ce que** l'on effectue l'opération de dilacération au moyen d'une pompe dilacératrice.

4. Procédé selon l'une quelconque des revendications précédentes, **caractérisé en ce que** les produits lourds sont issus de la fabrication du phénol, de préférence obtenu à partir de cumène.

5. Procédé selon l'une quelconque des revendications précédentes, **caractérisé en ce que** l'on met en contact les résidus, avant l'opération de dilacération, avec un mélange dont le point d'ébullition est supérieur à la température d'ébullition des résidus.

6. Procédé selon la revendication précédente, **caractérisé en ce que** le mélange précité est un sous-produit issu de la préparation du cumène.

7. Procédé selon l'une quelconque des revendications précédentes, **caractérisé en ce que** l'on effectue la réaction/distillation en présence d'un catalyseur.

8. Procédé selon la revendication précédente, **caractérisé en ce que** le catalyseur est choisi parmi l'acide sulfurique, les acides organiques sulfoniques.

9. Procédé selon l'une quelconque des revendications précédentes, **caractérisé en ce que** l'on neutralise les résidus après l'opération de dilacération, avant leur destruction.

10. Procédé selon l'une quelconque des revendications précédentes, **caractérisé en ce que** l'on effectue la distillation en présence d'un agent anti-mousse, de préférence choisi parmi les silicones.

11. Procédé selon l'une quelconque des revendications précédentes, **caractérisé en ce que** la réaction/distillation est effectuée dans des conditions telles que la température est comprise entre 200 et 300°C, de préférence entre 220 et 260°C.

12. Procédé selon l'une quelconque des revendications précédentes, **caractérisé en ce que** les composés sont neutralisés.

## Patentansprüche

1. Verfahren zur Rückgewinnung von in einem Gemisch von hochsiedenden Nebenprodukten enthaltenen Verbindungen, wobei man in dem Verfahren eine Reaktion/Destiliation durchführt, durch die man einerseits die genannten Verbindungen und andererseits Rückstände erhält, **dadurch gekennzeichnet, daß** man eine Zerkleinerung der Rückstände vor ihrer Zerstörung und bevor sie fest werden durchführt.

2. Verfahren nach dem vorangehenden Anspruch, **dadurch gekennzeichnet, daß** man den Zerkleinerungsvorgang nach Austritt aus der Reaktion/Destiliation durchführt.

3. Verfahren nach irgendeinem der vorangehenden Ansprüche, **dadurch gekennzeichnet, daß** man den Zerkleinerungsvorgang mit einer Zerkleinerungspumpe durchführt.

4. Verfahren nach irgendeinem der vorangehenden Ansprüche, **dadurch gekennzeichnet, daß** die hochsiedenden Nebenprodukte aus der Herstellung von Phenol hervorgegangen sind, das vorzugsweise aus Cumol entstanden ist.

5. Verfahren nach irgendeinem der vorangehenden Ansprüche, **dadurch gekennzeichnet, daß** man die Rückstände vor dem Zerkleinerungsvorgang mit einem Gemisch zusammengibt, dessen Siedepunkt über der Siedetemperatur der Rückstände liegt.

6. Verfahren nach dem vorangehenden Anspruch, **dadurch gekennzeichnet, daß** das genannte Gemisch ein Nebenprodukt ist, das aus der Herstellung von Cumol hervorgegangen ist.

7. Verfahren nach irgendeinem der vorangehenden Ansprüche, **dadurch gekennzeichnet, daß** man die Reaktion/Destillation in Gegenwart eines Katalysators durchführt.

8. Verfahren nach dem vorangehenden Anspruch, **dadurch gekennzeichnet, daß** der Katalysator aus Schwefelsäure und den organischen Sulfonsäuren gewählt ist.

9. Verfahren nach irgendeinem der vorangehenden Ansprüche, **dadurch gekennzeichnet, daß** man die Rückstände nach dem Zerkleinerungsvorgang und vor ihrer Zerstörung neutralisiert.

10. Verfahren nach irgendeinem der vorangehenden Ansprüche, **dadurch gekennzeichnet, daß** man die Destillation in Gegenwart eines Entschäumers durchführt, der vorzugsweise aus den Silikonen gewählt ist.

11. Verfahren nach irgendeinem der vorangehenden Ansprüche, **dadurch gekennzeichnet, daß** die Reaktion/Destillation unter Bedingungen durchgeführt wird, bei denen die Temperatur zwischen 200 und 300 °C, vorzugsweise zwischen 220 und 260 °C, liegt.

12. Verfahren nach irgendeinem der vorangehenden Ansprüche, **dadurch gekennzeichnet, daß** die Verbindungen neutralisiert sind.

## Claims

1. Process for the recovery of compounds contained in a mixture of heavy products, wherein a reaction/distillation is carried out, by means of which, on the one hand, the said compounds and, on the other hand, residues are obtained, **characterized in that** maceration of the residues is carried out before their destruction and before they set to a mass.

2. Process according to the preceding claim, **characterized in that** the maceration operation is carried out at the outlet of the reaction/ distillation.

3. Process according to one of the preceding claims, **characterized in that** the maceration operation is carried out by means of a macerator pump.

4. Process according to any one of the preceding claims, **characterized in that** the heavy products come from the manufacture of phenol, preferably obtained from cumene.

5. Process according to any one of the preceding claims, **characterized in that** the residues are, before the maceration operation, brought into contact with a mixture whose boiling point is higher than the boiling point of the residues.

6. Process according to the preceding claim, **characterized in that** the abovementioned mixture is a by-product coming from the preparation of cumene.

7. Process according to any one of the preceding claims, **characterized in that** the reaction/ distillation is carried out in the presence of a catalyst.

8. Process according to the preceding claim, **characterized in that** the catalyst is selected from sulphuric acid and organic sulphonic acids.

9. Process according to any one of the preceding claims, **characterized in that** the residues are neutralized after the maceration operation, before they are destroyed.

10. Process according to any one of the preceding claims, **characterized in that** the distillation is carried out in the presence of an antifoaming agent, preferably selected from among silicones.

11. Process according to any one of the preceding claims, **characterized in that** the reaction/ distillation is carried out under such conditions that the temperature is between 200 and 300°C, preferably between 220 and 260°C.

12. Process according to any one of the preceding claims, **characterized in that** the compounds are neutralized.
